(19) Europäisches Patentamt
European Patent Office
Office européen des brevets

(11) **EP 2 942 630 B1**

(12) **EUROPEAN PATENT SPECIFICATION**

(45) Date of publication and mention
of the grant of the patent:
**17.10.2018 Bulletin 2018/42**

(51) Int Cl.:
*G01N 33/574* *(2006.01)*     *G01N 33/84* *(2006.01)*

(21) Application number: **15000609.6**

(22) Date of filing: **03.03.2015**

(54) **DIAGNOSTIC METHOD WITH USE OF INDICATING ELEMENTS (YVS' METHOD)**

DIAGNOSEVERFAHREN MIT ANZEIGEELEMENTEN (YVS-VERFAHREN)

PROCÉDÉ DE DIAGNOSTIC AVEC UTILISATION D'ÉLÉMENTS D'INDICATION (PROCÉDÉ YVS)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.05.2014 RU 2014118321**

(43) Date of publication of application:
**11.11.2015 Bulletin 2015/46**

(73) Proprietor: **Sokolov, Yuri Vladimirovich**
**105062 Moscow (RU)**

(72) Inventor: **Sokolov, Yuri Vladimirovich**
**105062 Moscow (RU)**

(74) Representative: **Zielinski, Wojciech Leszek**
**Wojciech Zielnski - Uslugi Projektowe I Prace Innowacyjne**
**ul. Armii Polskiej 18/5**
**66-400 Gorzow Wielkopolski (PL)**

(56) References cited:
• **CLARISSA GOMES ET AL: "TRACE ELEMENT ANALYSIS OF PROTEINS DIRECTLY FROM 2D-PAGE: AN EFFICIENT STRATEGY FOR METALLOPROTEOMICS", PREPARATIVE BIOCHEMISTRY AND BIOTECHNOLOGY, vol. 41, no. 3, 1 July 2011 (2011-07-01), pages 236-242, XP055197749, ISSN: 1082-6068, DOI: 10.1080/10826068.2011.575272**

## Description

The invention relates to the medicine and consists in identifying a designated cluster of indicator elements that differentiate patients into those who are diagnosed with diseases of various etiologies (cardiovascular or cancer cases) and those who are presumed to be healthy.

According to the statistics available at the Russian Ministry of Health for 2012, people who have been originally diagnosed with a malignancy is 480 thousand, while the total number of patients with this diagnosis is 1,656 thousand, which is 335.2 cases per 100,000 population.

The one-year mortality rates in patients with malignancies in the Russian Federation for 2012 (who died within the first year of being diagnosed in %), including according to the nature of medical condition:

- Esophageal cancer - 59.4%
- Lung cancer - 52.4%
- Gastric cancer - 49.8 %
- Rectal cancer - 25.8%
- Lymphatic and hematopoietic cancer - 21.7%
- Cervical cancer - 17.0%
- Breast cancer - 8.3%

These figures are testament to poor quality and delayed diagnosis. Diagnoses are made when treatment is difficult, mostly altogether expensive, and completely hopeless. Every third cancer patient dies in Russia within a year after being diagnosed, although cancer has been fairly inexpensively and effectively treated in the civilized world for many years.

**Cardiovascular diseases (CVD) are the leading cause of death in most developed countries, including Russia.**

CVD can well be called the number one problem of the present-day Russia since the cardiovascular mortality rate statistics are extremely disappointing: 57% of deaths in the total mortality in the Russian Federation are attributed to cardiovascular diseases. During the first 9 months of 2013, the cardiovascular disease related death rate reached about 1 million cases. And, even though according to Rosstat (the Federal State Statistics Service), the tally of deaths resulting from circulatory system diseases dropped 19.1% from 2003 to 2011, fighting this life-threatening disease remains an overarching challenge.

A number of factors cause changes in the health status and these changes can be irreversible, and thus lead to diseases of various etiology. To date, these processes have not been completely identified. However, there exist such instruments as indication or reference markers making it possible to identify diseases. The medical literature does not give due attention to the study of chemical elements in the human body that as is commonly known constitute all living things on Earth. Research work is underway on diagnosing cancer diseases of various etiologies at the genetic level, but all those methods are at the initiation stage and are developed on a limited scale in Europe.

The invention is based on long-term research effort of the element blood composition of people affected by various diseases and people presumed to be health.

The method for trace element analysis (see "Method for Trace Element Analysis in Diagnosed Biosubstrates Employing Inductively Coupled Plasma Mass Spectrometry (ICP-MS; Guidelines" approved by the Russian Ministry of Health 26.03.2003)).

However, the above technique does provide any insight as to how obtained data on the element composition of a blood sample can be used for diagnosis of a human disease.

The technical result that the invention is intended to achieve is the ability to diagnose cardiovascular and cancer diseases in humans by identifying specific elements in a blood sample.

The technical result is achieved by a disease diagnosis method using indicator elements to examine the element composition of a blood sample used for diagnosis based on the presence of chemical elements in a blood sample Cr, Eu, Tb, Er, Tm, Lu, Ta, Ba, V, Pd having contents as detailed below

Cr below $0.0119 \pm 0.010$ mg/kg
Eu below $4.33 \ast 10^{-5} \pm 8.57 \ast 10^{-6}$ mg/kg
Tb below $2.06 \ast 10^{-5} \pm 9.06 \ast 10^{-6}$ mg/kg
Er below $2.27 \ast 10^{-5} \pm 9.96 \ast 10^{-6}$ mg/kg
Tm below $1.31 \ast 10^{-5} \pm 9.85 \ast 10^{-6}$ mg/kg
Lu below $1.25 \ast 10^{-5} \pm 1.04 \ast 10^{-5}$ mg/kg
Ta below $2.31 \ast 10^{-5} \pm 2.11 \ast 10^{-10}$ mg/kg

Ba above 0.0152±0.009 mg/kg
v above 0.0122±0.079 mg/kg
Pd above 0.000187+0.00013 mg/kg

The index value is calculated as follows:

$$Y = (10^{-3}*3*Cr + 5* (Eu+Tb +Er+Tm+ Lu+Ta))/(10^{-1}*(V + 4*Ba)+5*Pd).$$

Where the index value Y is greater than 0.093, this indicates the absence of cardiovascular or cancer diseases.

Where the value index Y is below 0.026, a cardiovascular disease or a type of cancer pathology is diagnosed. The index Y indicates an individual's health status only from the age of 19.

Blood sampling and sample preparation technique

A blood sample was collected by medical personnel using a 5 ml syringe from the antecubital vein into tubes containing anticoagulant special ethylenediaminetetraacetic acid (EDTA) that prevents blood clotting. 1-2 ml of blood is the sufficient quantity for element composition analysis.

The following parameters have been taken into account in sampling:

- age (including the birth date where possible),
- sex,
- disease (if any),
- blood sample,
- Rh factor

The number of blood samples collected from people of different age groups (from 0 to 98 years) totaled above 3,000, including: blood samples from people diagnosed with malignancies of different etiology and cardiovascular diseases. The overall sampling can be assumed to be statistically meaningful and representative.

The age groups isolated for blood sampling purposes are: group 1 - 0-10 years, group 2 - 11-20 years, group 3 - 21-30 years, group 4 - 31-40 years, group 5 - 41-50 years, group 6 - 51-60 years, group 7 - 61-70 years, group 8 - 71-80 years, group 9 - 81-90 years, group 10 - 91-100 years.

Bloods samples were prepared by acid decomposition in a closed pressure tank as required by MUK guidelines 4.1.148-03. Below is the procedure for preparing blood samples for analysis.

1. Blood sample preparation procedure

1.1. Place 0.5-1.2 g of a blood sample in a 50 ml plastic tube. Add 2.5 ml of 70% vol. nitric acid and 250 μl of a 30% vol. hydrogen peroxide solution and thoroughly mix to dissolve the clot.

1.2. Put the contents of the test tubes into the pressure tank.

1.3. Add 1 ml of 70% vol. nitric acid to the tube. Wash away from the sample residue off the tube walls. Dissolve if necessary any sample residue left on the tube walls by heating the tube in a microwave oven.

1.4. Put the contents of the test tubes into the appropriate pressure tank.

1.5. Shut the inner cover in the pressure tank, putting a safety aluminum disc atop the cover and screw down the top cover.

1.6. Place the pressure tanks into the Speed E microwave in the order prescribed by the manufacturer.

1.7. Activate the 4th decomposition program comprising the following steps:

1.7.1. Heating the sample to 140°C for 10 min.

1.7.2. Incubating at 140°C for 5 min.

1.7.3. Heating the sample to 200°C for 2 min.

1.7.4. Incubating at 200°C for 15 min.

1.8. Cool down the pressure tanks to a temperature not higher than 50 °C.

1.9. Slowly open the cooled pressure tank preventing the loss of fluid entrapped by the nitric acid vapor.

1.10. Transfer the contents of the pressure tank in a 50 ml plastic test tube.

1.11. Add 20 ml of 1% vol. nitric acid into the pressure tank to create a sample weighing ≤ 0.6 g or 1 ml of 45% nitric acid for sample weight> 0.6 g

1.12. Pour the contents of the pressure tank into an appropriate plastic tube. Increase the volume of deionized water to 25 ml to create a sample weighing ≤ 0.6 g or to 50 ml to create a sample weighing > 0.6 g.

1.13. Mix thoroughly.

1.14. Add a recovery standard 50 μl solution (10ppb In, Rh) to each tube containing a 50 ml sample solution. Add a recovery standard 25 μl solution (10ppb In, Rh) to each tube containing a 25 ml sample solution.

1.15. Mix thoroughly.

Rhodium (Rh) was used as a recovery standard.

Once samples had been prepared, measurements were made using an Elan DRC-e inductively coupled plasma mass spectrometer.

So, a high-quality highly sensitive analysis, inductively coupled plasma mass spectrometry (ICP-MS), that was made on the inductively coupled plasma mass spectrometer Elan Model 9000, DRC-e (registration number No. 26441-04 dated 23.09.2009) was used as a research method.

The inductively coupled plasma mass spectrometry method is capable of identifying to determine the contents of the following elements: Li, Be, B, Na, Mg, Al, Si, P, S, K, Ca, Ti, V,Cr, Mn, Fe, Co, Ni, Cu, Zn, Ga, Ge, As, Se, Br, Rb, Sr, Y, Zr, Nb, Mo, Pd, Ag, Cd, Sn, Sb, Te, I, Cs, Ba, La, Ce, Pr, Nd, Sm, Eu, Gd, Tb, Dy, Ho, Er, Tm, Yb, Lu, Hf, Ta, W, Re, Ir, Pt, Au, Hg, Tl, Pb, Bi, Th, U.

The certified blood standard (trace amounts of elements in blood, level 2 (Seronorm T.Elem.Whole Blood L-2) was used as a measurement monitoring control

In the course of the research groups of elements in blood samples of people affected with diseases and of people presumed to be healthy have behaved differently. We have carried out strict indicator differentiation of diseases.

Using information on the content of a certain element - an indicator (Cr, Eu, Tb, Er, Tm, Lu, Ta) may be testament to the presence of a disease, e.g. in the blood of an individual presumed to be healthy Cr, Eu, Tb, Er, Tm, Lu, Ta is higher than that an individuals affected with a malignancy or a cardiovascular problem, while the content of Ba, V, Pd in the blood of people affected with diseases is higher in comparison with that found in an individual presumed to be healthy.

The most common malignancies are singled out and studied in detail:

- lung cancer;
- gastric cancer;
- breast cancer;
- prostate cancer.

The most common cardiovascular diseases are singled out and studied in detail:

- hypertensive disease;
- coronary heart disease;
- heart stroke;
- heart attack

1. The research project is conducted to determine the content of indicator parameters (Cr, Eu, Tb, Er, Tm, Lu, Ta, Ba, V, Pd) in the blood sample using inductively coupled plasma mass spectrometry (ICP-MS);

2. Set out the range of indication values for a tested blood sample.

Specify the values of the indicator elements in the blood of people from different groups (mg/kg)

| Chemical elements | Presumably healthy | Cardiovascular group | Oncology |
|---|---|---|---|
| Cr | $0.0119 \pm 0.010*$ | $0.00374 \pm 0.0016$ | $0.00389 \pm 0.0018$ |
| Eu | $4.33*10^{-5} \pm 8.57*10^{-6}$ | $1.02*10^{-5} \pm 9.72*10^{-6}$ | $1.26*10^{-5} \pm 9.06*10^{-6}$ |
| Tb | $2.06*10^{-5} \pm 9.06*10^{-6}$ | $4.75*10^{-6} \pm 2.87* 10^{-6}$ | $4.51*10^{-6} \pm 1.57*10^{-6}$ |
| Er | $2.27*10^{-5} \pm 9.96*10^{-6}$ | $1.18*10^{-5} \pm 9.61*10^{-6}$ | $1.14*10^{-5} \pm 8.51 *10^{-6}$ |
| Tm | $1.31*10^{-5} \pm 9.85*10^{-6}$ | $4.28*10^{-6} \pm 1.89*10^{-6}$ | $4.39*10^{-6} \pm 1.64*10^{-6}$ |
| Lu | $1.25*10^{-5} \pm 1.04*10^{-5}$ | $5.38*10^{-6} \pm 4.19*10^{-6}$ | $4.52*10^{-6} \pm 2.31*10^{-6}$ |

(continued)

| Chemical elements | Presumably healthy | Cardiovascular group | Oncology |
|---|---|---|---|
| Ta | $2.31*10^{-5}\pm2.11*10^{-5}$ | $7*10^{-6}\pm3.26*10^{-6}$ | $5.45*10^{-6}$ $\pm2.42*10^{-6}$ |
| V | $0.0122\pm0.0079$ | $0.0215\pm0.0095$ | $0.0188\pm0.0054$ |
| Pd | $0.000187\pm0.00013$ | $0.000282\pm0.00011$ | $0.000283\pm0.00010$ |
| Ba | $0.0152\pm0.009$ | $0.0623\pm0.041$ | $0.0841\pm0.055$ |

3. The index value is calculated as follows: $Y = (10^{-3}*3*Cr + 5*(Eu+Tb +Er+Tm+ Lu+Ta))/ (10^{-1}*(V + 4*Ba)+5*Pd)$. Based on calculated values of the index, diseases of various etiologies are diagnosed, for example, cancer or cardiovascular diseases where the index value Y is below 0.026.

**Claims**

1.  A diagnosis method using indicator elements to examine the element composition of a blood sample used for diagnosis based on the presence of chemical elements in a blood sample that differ in patients aged above 18 with the contents of Cr, Eu, Tb, Er, Tm, Lu, Ta, Ba, V, Pd as detailed below

    Cr below $0.0119\pm0.010$ mg/kg
    Eu below $4.33*10^{-5}\pm8.57*10^{-6}$ mg/kg
    Tb below $2.06*10^{-5}\pm9.06*10^{-6}$ mg/kg
    Er below $2.27*10^{-5}\pm9.96*10^{-6}$ mg/kg
    Tm below $1.31*10^{-5}\pm9.85*10^{-6}$ mg/kg
    Lu below $1.25*10^{-5}\pm1.04*10^{-5}$ mg/kg
    Ta below $2.31*10^{-5}\pm2.11*10^{-5}$ mg/kg
    Ba above $0.0152\pm0.009$ mg/kg
    V above $0.0122\pm0.079$ mg/kg
    Pd above $0.000187\pm0.00013$ mg/kg

    The index value is calculated as follows:
    $Y = (10^{-3}*3*Cr+5*(Eu+Tb+Er+Tm+ Lu+Ta))/(10^{-1}*(V+4*Ba)+5*Pd)$ with a cardiovascular disease or a cancer pathology being diagnosed where Y is below 0.026 and no medical problem is identified where Y is above 0.093.

**Patentansprüche**

1.  Diagnoseverfahren unter Verwendung von Indikatorelementen zur Untersuchung der chemischen Zusammensetzung von Blutproben. Es wird für Diagnosen auf Grundlage des Gehalts an chemischen Elementen in Blutproben verwendet, das sich bei Patienten in einem Alter über 18 Jahre unterscheidet und die Elemente Cr, Eu, Tb, Er, Tm, Lu, Ta , Ba, V, Planungsdokumentation betrifft, wie nachfolgend aufgeführt:

    Cr unterhalb $0,0119\pm0,010$ mg/kg
    Eu unterhalb $4,33*10^{-5}\pm8,57*10^{-6}$ mg/kg
    Tb unterhalb $2,06*10^{-5}\pm9,06*10^{-6}$ mg/kg
    Er unterhalb $2,27*10^{-5}\pm9,96*10^{-6}$ mg/kg
    Tm unterhalb $1,31*10^{-5}\pm9,85*10^{-6}$ mg/kg
    Lu unterhalb $1,25*10^{-5}\pm1,04*10^{-5}$ mg/kg
    Ta unterhalb $2,31*10^{-5}\pm2,11*10^{-5}$ mg/kg
    Ba über $0,0152\pm0,009$ mg/kg
    V über $0,0122\pm0,079$ mg/kg
    Pd über $0,000187\pm0,00013$ mg/kg

Der Wert der Kennziffer wird auf die nachfolgend angegebene Weise berechnet:

$$Y = (10^{-3}*3*Cr+5*(Eu+Tb+Er+Tm+Lu+Ta))/(10^{-1}*(V+4*Ba)+5*Pd),$$

wobei ein Wert der Kennziffer Y unterhalb von 0,026 auf Krankheiten des Kreislaufsystems oder einen Tumor hinweist, ein Wert über 0,093 dagegen auf das Fehlen gesundheitlicher Probleme.

**Revendications**

1. Un procédé de diagnostic utilisant des éléments indicateurs pour examiner la composition d'éléments dans un échantillon de sang utilisé pour le diagnose en fonction de la présence d'éléments chimiques qui se trouve dans le sang et qui diffèrent chez les patients âgés de plus de 18 ans avec les contenus Cr, Eu, Tb, Er, Tm, Tm, Lu, Ta, Ta, Ba, V, Pd comme détaillé ci-dessous :

   Cr inférieur à $0.0119 \pm 0.010$ mg/kg
   Eu inférieur à $4.33*10^{-5} \pm 8.57*10^{-6}$ mg/kg
   Tb inférieur à $2.06*10^{-5} \pm 9.06*10^{-6}$ mg/kg
   Er inférieur à $2.27*10^{-5} \pm 9.96*10^{-6}$ mg/kg
   Tm inférieur à $1.31*10^{-5} \pm 9.85*10^{-6}$ mg/kg
   Lu inférieur à $1.25*10^{-5} \pm 1.04*10^{-5}$ mg/kg
   Ta inférieur à $2.31*10^{-5} \pm 2.11*10^{-5}$ mg/kg
   Ba supérieur à $0.0152 \pm 0.009$ mg/kg
   V supérieur à $0.0122 \pm 0.079$ mg/kg
   Pd supérieur à $0.000187 \pm 0.00013$ mg/kg

   La valeur index est calculée comme suit :
   $Y = (10^{-3}*3*Cr+5*(Eu+Tb+Er+Tm+Lu+Ta)/(10^{-1}*(V+4*Ba)+5*Pd)$ avec une maladie cardiovasculaire ou un cancer étant diagnostiquée, où Y est inférieur à 0,026 et aucun problème médical n'a été identifié lorsque Y est supérieur à 0,093.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Method for Trace Element Analysis in Diagnosed Biosubstrates Employing Inductively Coupled Plasma Mass Spectrometry (ICP-MS; Guidelines. Russian Ministry of Health, 26 March 2003